# EUROPEAN PATENT SPECIFICATION

(11) **EP 1 327 885 B1**
(45) Date of publication and mention of the grant of the patent: **09.03.2005**
(21) Application number: 01274437.1
(22) Date of filing: 30.10.2001
(51) Int. Cl.: G01N 33/544, G01N 33/571, G01N 33/576

(54) **A KIT FOR THE SIMULTANEOUS DIAGNOSIS OF A PLURALITY OF INFECTIOUS DISEASES AND A METHOD FOR PREPARATION OF IT**
KIT ZUR SIMULTANEN DIAGNOSE MEHRERER ANSTECKENDER KRANKHEITEN UND VERFAHREN ZU SEINER HERSTELLUNG
KIT DE DIAGNOSTIC SIMULTANE D'UNE PLURALITE DE MALADIES INFECTIEUSES ET TECHNIQUE DE PREPARATION DE CELUI-CI

(30) Priority: 17.08.2001 CN 01126500
(43) Date of publication of application: 16.07.2003
(73) Proprietor: Shanghai Health Digit Limited, 200233 Shanghai (CN)
(72) Inventor: HU, Gengxi, 200233 Shanghai (CN)
(74) Representative: Hinterberg, Katherine
(86) International application number: PCT/CN2001/001519
(87) International publication number: WO 2003/016914

(56) References cited:
- EP-A- 0 302 154
- WO-A-00/00830
- WO-A-91/01003
- CN-A- 1 140 258
- CN-A- 1 307 238
- JP-A- 2000 097 942
- US-A- 5 637 508
- IJSSELMUIDEN O E ET AL: "OPTIMIZING THE SOLID-PHASE IMMUNOFILTRATION ASSAY A RAPID ALTERNATIVE TO IMMUNOASSAYS" JOURNAL OF IMMUNOLOGICAL METHODS, ELSEVIER SCIENCE PUBLISHERS B.V.,AMSTERDAM, NL, vol. 119, no. 6, 1989, pages 35-43, XP009010519 ISSN: 0022-1759
- HUANG QIAOJIA LAN XIAOPENG ET AL: "Dot-immunogold filtration assay as a screening test for syphilis" JOURNAL OF CLINICAL MICROBIOLOGY, vol. 34, no. 8, 1996, pages 2011-2013, XP001179724 ISSN: 0095-1137

## Description

### Field of the invention

This invention relates to the field of biotechnology, more particularly, to a diagnostic kit for simultaneously detecting multiple infectious diseases and the preparation thereof.

### Background of the invention

Hepatitis B, Hepatitis C, syphilis and AIDS are four kinds of worldwide infectious diseases. According to the statistical materials from Ministry of Health, the incidence of these diseases occupies about 27.57% among the total incidence of infectious diseases, which does great harm to the public and people's living.

Hepatitis B is an inflammation of the liver which may be caused by drugs, toxin and virus. About 10%-15% of the population is infected with hepatitis b virus (HBV) in China. HBV is transmitted through transfusion, plasma, blood products or by contacting the injection needles, syringes and tools for blood sampling and haemodialysis. Among them, the infection caused by transfusion has been paid great attention to since it is high in incidence and develops rapidly.

The origin of the infection of Hepatitis C mainly comprises acute clinical patients, non-symptom subclinical patients, chronic patients and virus carriers. The virus can be detected in the blood before 12 days of the attack of the disease, and will remain for more than 12 years. The infection of hepatitis C is mainly through transfusion route. In most of the developed countries, hepatitis C is one of the common types of the hepatitis infected after transfusion. It is estimated that 30-90% of cases of hepatitis infected after transfusion are hepatitis C in foreign countries, while in China, about one third.

Syphilitic patients are the only origin of infection of treponema pallidum. About 95% of the transmission of syphilis is through sexual contact. Syphilis also can be transmitted from the donor to the acceptor through transfusion. Syphilis will be transmitted when the donor suffering from syphilis is in the stage of treponemapallidumia. Treponema pallidum has a low viability in vitro, existing for 48-72 hours at 4°C, having no infectivity at 40°C, and dying immediately at 100°C. In China, the cases of venereal disease are increasing in recent years. Therefore, close attention should be paid to the prevention of the transmission of syphilis through transfusion.

HIV is the pathogen of AIDS and is mainly transmitted through blood, spermatic fluid, vaginal secreta and breast milk. The possibility of the infection of HIV will exceed 90% if the blood with HIV is transfused. On the contrary, the risk is less than 1% through sexual intercourse. In addition, the transfusion imports a large amount of HIV. It will develop to AIDS rapidly during 3-5 years (about 2 years for children).

Therefore, it is necessary to examine strictly the virus in blood or blood products which can transmit diseases through transfusion, including HIV, syphilis, HBV and HCV. The Ministry of Health of China has stipulated, in "the standards for examining the health of the blood donors" published in 1998, nine items which should be detected, including hepatitis B virus surface antigen (HBsAg), HCV antibody, HIV antibody and syphilitic antibody. At present, RPR (rapid plasma reagin) method or TRUST method (serological test for syphilis) is used to detect syphilis. ELISA (enzyme linked immune absorbent assay) is used to detect HIV, HBV and HCV. This method needs 400ul of serum per one examination and takes 1-2 hours. Many people are infected with the infectious disease such as hepatitis, AIDS and syphilis through transfusion due to the lack of a method for rapidly, completely and simultaneously detecting multiple diseases as to the blood of the donor. This greatly influences the health of the donee and the stability of the public.

Colloidal immunogold filtration assay is based on ELISA technique. Initially, the conjugates were labeled with enzyme and the method was called enzyme immune filtration assay. At the beginning of 1990's, colloidal gold was adopted as the label and the method was called immunogold filtration assay (IGFA).

The principle of the immunogold filtration assay is as follows. By selecting nitrocellulose membrane as the carrier and employing the capillary action and permeability of the millipore membrane, the antigens and the antibodies are reacted and washed on a special filtration device. The reaction is completed rapidly in the manner of liquid filtration through membrane.

The method has been widely used clinically because it is simple and rapid, does not need any instruments except the reagents, and the results can be observed by the naked eye in several minutes. This method can be used in every field of immune detection, especially for detecting antigenic substances not existing in normal body fluid, e.g. antigens or antibodies in infectious disease, and the substances, e.g. HCG, alpha fetoprotein, the amounts of which are normally low but increases abnormally under special conditions. With the selection of materials and the improvements of preparation techniques, the application of this method became wider. However, the colloidal immunogold filtration assay for simultaneously detecting the antigens or antibodies of multiple infectious diseases still has not been reported yet.

### Summary of the invention

It is an object of the present invention to provide a sensitive, accurate and rapid diagnostic multiplepurpose kit for simultaneously detecting multiple infectious diseases utilizing colloidal immunogold filtration assay technique.

According to the present invention, the diagnostic kit for simultaneously detecting multiple infectious diseases includes an immunogold filtration assay device, buffer and the mixed solution of colloidal gold conjugates, wherein said immunogold filtration assay device comprises a nitrocellulose membrane dotted thereon separately by HBsAg monoclonal antibody (mouse anti-human), HCV antigen, syphilitic antigen, HIV antigen and goat anti-mouse IgG antibody for quality control; the volume of each dotted sample is 0.3-3 µl.

Said mixed solution of colloidal gold conjugates comprises four kinds of colloidal gold conjugates, including the colloidal gold-labeled HBsAg monoclonal antibody (mouse anti-human), HCV antigen, syphilitic antigen and HIV antigen; wherein the concentration of the colloidal gold-labeled HBsAg monoclonal antibody is 20-50 µg/ml, the concentration of the colloidal gold-labeled HCV antigen is 90-120 µg/ml, the concentration of the colloidal gold-labeled syphilitic antigen is 90-120µg/ml and the concentration of the colloidal gold-labeled HIV antigen is 80-120 µg/ml. Said colloidal gold particles have the size of 20-30 nm and these four kinds of conjugates are mixed in the ratio of 1:1:1.2-2.5:1.2-2.5.

Said buffer comprises 0.03-0.05% Tween 20 and PBS buffer (pH 7.2-8.8, which is prepared from potassium dihydrogen phosphate or from sodium dihydrogen phosphate and disodium hydrogen phosphate).

According to the present invention, said syphilitic antigen is a single syphilitic antigen or the mixture of multiple syphilitic antigens. Said HIV antigen is HCV-1 (I type HIV) antigen, HIV-2 (II type HIV) antigen or the mixture of HIV-1 antigen and HIV-2 antigen.

Another object of the present invention is to provide a method for preparing the said diagnostic kit.

According to the present invention, the method for preparing the diagnostic kit for simultaneously detecting multiple infectious diseases comprises the following steps:
Step A). preparing the immunogold filtration assay device, which comprises the following steps:
   1). preparing the samples applied on the membrane
      The HBsAg monoclonal antibody (mouse anti-human) is dissolved with PBS (0.02M, pH8.0) and dialyzed overnight at 4°C. Then, the HBsAg monoclonal antibody is diluted to 0.2-2.0 mg/ml with PBS (0.02M, pH8.0) and stored at 4°C for further use.
      The HCV antigen is dissolved with PBS (0.02M, pH8.0) and dialyzed overnight at 4 °C. Then the HCV antigen is diluted to 0.2-2.0 mg/ml with PBS (0.02M, pH8.0) and stored at 4°C for further use.
      The syphilis antigen is dissolved with PBS (0.02M, pH8.0) and dialyzed overnight at 4°C. Then the syphilis antigen is diluted to 0.2-2.0 mg/ml with PBS (0.02M, pH8.0) and stored at 4°C for further use.
      The HIV antigen is dissolved with PBS (0.02M, pH8.0) and dialyzed overnight at 4°C. Then the HIV antigen is diluted to 0.2-2.0mg/ml with PBS (0.02M, pH8.0) and stored at 4 °C for further use.
      The goat anti-mouse IgG antibody is dissolved with PBS (0.02M, pH8.0) and dialyzed overnight at 4 °C. Then, the goat anti-mouse IgG antibody is diluted to 0.2-2.0mg/ml with PBS (0.02M, pH8.0) and stored at 4°C for further use.
   2). dotting the samples onto the membrane
      The above described HBsAg monoclonal antibody, HCV antigen, syphilitic antigen, HIV antigen are pipetted and carefully dotted onto the specific positions on the nitrocellulose membrane in a volume of 0.3-3µl for each protein.
      Additionally, the goat anti-mouse IgG antibody is pipetted for labeling a mark at a position far from the above dotting position on the nitrocellulose membrane. Said mark may be a dot, a line or in some other forms.
   3). post-treating the membrane
      The above nitrocellulose (NC) membrane is dried in an oven for 30 minutes at 37°C and left at room temperature for over 20 minutes. Then, the membrane is immersed in a blocking solution at 37°C for 20 minutes and washed and vibrated in a washing solution for 5-10 minutes at room temperature. The above washing step is repeated after changing the washing solution. Then, the membrane is taken out, air dried at room temperature, and stored at 4°C under dry environment for further use.
      Said blocking solution comprises 0.01-0.03% Tween 20 and 0.05M PBS buffer (pH7.2-8.0). Said washing solution comprises 0.03-0.05% Tween 20 and 0.01M PBS buffer (pH7.2-8.0).
   4). assembling the device

   Two layers of water-absorbing filter paper are laid under the nitrocellulose membrane. Both the nitrocellulose membrane and the filter paper are placed and fixed in the plastic reaction device.
Step B). preparing the mixed solution of the colloidal gold conjugates, which comprises the following steps:
   1). preparing the colloidal gold particles
      0.01% chloroauric acid solution is heated to boiling point and 1% sodium citrate solution is added quickly. The solution is kept boiling for 5 minutes to make the size of the colloidal gold particles to be 20-30nm.
   2). preparing the colloidal gold conjugates
   2.1). preparing the colloidal gold-labeled HBsAg monoclonal antibody (mouse anti human)
      The HBsAg monoclonal antibody is slowly added into the colloidal gold solution, of which the gold particle diameter is 20-30nm, under magnetic stirring to a final concentration of 20-50µg/ml. After stirring for 30 minutes at room temperature, 10% BSA is added to make the antibody to a final concentration of 0.2-1.0% and the mixture is stirred for 5 minutes at room temperature. 10% PEG20000 solution is added to make the antibody to a final concentration of 0.1-0.5% and the mixture is stirred for 5 minutes at room temperature. The mixture is centrifuged at 12000-15000r/min for 60 minutes. The supernatant is discarded and the precipitate is dissolved in a storing solution. The solution is filtered through a 0.45 µm filter membrane and stored at 4°C for further use.
   2.2). preparing the colloidal gold-labeled HCV antigen
      The HCV antigen is slowly added into the colloidal gold solution of which the gold particle diameter is 20-30nm, under magnetic stirring to a final concentration of 90-120µg/ml. After stirring for 30 minutes at room temperature, 10% BSA solution is added to make the antigen to a final concentration of 0.2-1.0% and the mixture is stirred for 5 minutes at room temperature. Then, 10% PEG20000 solution is added to make the antigen a final concentration of 0.1-0.5% and the mixture is stirred for 5 minutes at room temperature. The mixture is centrifuged at 12000-15000 r/min for 60 minutes. The supernatant is discarded and the precipitate is dissolved in a storing solution. The solution is filtered through a 0.45 µm filter membrane and stored at 4°C for further use.
   2.3). preparing the colloidal gold-labeled syphilitic antigen
      The syphilitic antigen is slowly added into the colloidal gold solution of which the gold particle diameter is 20-30nm, under magnetic stirring, to a final concentration of 90-120 µg/ml. After stirring for 30 minutes at room temperature, 10% BSA solution is added to make the antigen a final concentration of 0.2-1.0% and the mixture is stirred for 5 minutes at room temperature. Then, 10% PEG20000 solution is added to make the antigen a final concentration of 0.1-0.5% and the mixture is stirred for 5 minutes at room temperature. The mixture is centrifuged at 12000-15000 r/min for 60 minutes. Then, the supernatant is discarded and the precipitate is dissolved in a storing solution. The solution is filtered through a 0.45 µm filter membrane and stored at 4°C for further use.
      When the syphilitic antigen in the colloidal gold-labeled solution is the mixture of multiple syphilitic antigens, each colloidal gold-labeled syphilitic antigens should be prepared separately, then mixed and stored for further use.
   2.4). preparing the colloidal gold-labeled HIV antigen
      The HIV antigen is slowly added into a colloidal gold solution of which the gold particle diameter is 20-30nm, under magnetic stirring, to a final concentration of 80-120 µg/ml. After stirring for 30 minutes at room temperature, 10% BSA solution is added to make the antigen a final concentration of 0.2-1.0% and the mixture is stirred for 5 minutes at room temperature. Then, 10% PEG20000 solution is added to make the antigen a final concentration of 0.1-0.5% and the mixture is stirred for 5 minutes at room temperature. The mixture is centrifuged at 12000-15000 r/min for 60 minutes. The supernatant is discarded and the precipitate is dissolved in a storing solution. The solution is filtered through a 0.45 µm filter membrane and stored at 4°C for further use.
      When the HIV antigen in the colloidal gold-labeled solution is the mixture of multiple HIV antigens, each colloidal gold-labeled HIV antigens should be prepared separately, then mixed and stored for further use.
   3). preparing the mixed solution of the colloidal gold conjugates

   The separately prepared colloidal gold conjugates are mixed with the volume ratio of the HBsAg monoclonal antibody : HCV antigen : syphilitic antigen : HIV antigen being 1:1:1.2-2.5:1.2-2.5.
Step C. preparing the buffer
   Tween 20 is added into PBS (pH7.2-8.8) to a final concentration of 0.03-0.05%.
   Another object of this invention is to provide the use of the above diagnostic kit for simultaneously detecting Hepatitis B, Hepatitis C, syphilis, and AIDS.

The detection method of the kit according to the present invention comprises the following steps:1. placing horizontally the immunogold filtration assay device on the desk;
2. dripping two drops of buffer into the sample well of the device to wet the surface of the membrane;
3. adding 50µl sample serum into the sample well, wait, until it has been completely absorbed.
4. adding three drops of buffer and after it has been completely absorbed adding two drops of colloidal gold-labeled solution;
5. adding three drops of buffer;
6. one minute later, observing whether there are red dots in the sample well, wherein red dot denotes the result of corresponding virus being positive while colorless one denotes negative result.

When the sample serum, which may contain one or several of HBsAg, HCV antibody, syphilis antibody and HIV antibody, contacts the nitrocellulose membrane, the antigen or antibody in the serum will react rapidly with its counterpart on the membrane. Then, the complex will react with the corresponding colloidal gold conjugates and form color. Therefore, the virus infected the patients can be judged based on the color. The goat anti-mouse IgG antibody can not react with the antibody in human sera, while the HBsAg monoclonal antibody (mouse anti-human) in the colloidal gold-labeled solution can react with said goat anti-mouse antibody. Therefore, color will appear at the corresponding position of the goat anti-mouse antibody, which may be recognized by the naked eye. When the kit can not be used for detection due to quality problems of certain components, color will not appear at the position corresponding to the goat anti-mouse IgG. Therefore, the goat anti-mouse IgG antibody serves as the quality control for the diagnostic kit.

The present invention, which based on the immunogold filtration assay technique, makes the corresponding antigens or antibodies of multiple infectious diseases react and wash in the same filtration device (nitrocellulose membrane), multiple infectious diseases can be detected by observing the colors of each dot by the naked eye.

Compared with the traditional gold-labeled kit, the PRP diagnostic kit and the TRUST diagnostic kit, the diagnostic kit of the present invention has the following advantages:
(1) simultaneously detecting multiple diseases
   The kit of the present invention can simultaneously detect four kinds of infectious diseases in one device.
(2) simultaneously detecting multiple antigens and antibodies

It can simultaneously detect multiple antigens or antibodies in one device, realizing the integration of the detection conditions of different proteins.

Compared with ELISA diagnostic kit, the diagnostic kit of the present invention has the following advantages:
(1) simultaneously detecting multiple diseases
   The kit of the present invention can simultaneously detect four kinds of infectious diseases in one device.
(2) simultaneously detecting multiple antigens and antibodies
   It can simultaneously detect multiple antigens or antibodies in one device, realizing the integration of the detecting conditions of different proteins.
(3) rapid reaction
   The whole assay comprises only two steps: adding the samples and reacting, which is achieved by filtration, and requires only 3-5 minutes. It is suitable for large scale blood screens. For the whole detection process only takes several minutes. However, the sensitivity is the same as ELISA, which, requires 1-2 hours for reaction.
(4) little requirement of sample blood
   The kit of the present invention requires only 50 µl of serum, which can be obtained from finger tips or earlobes. However, ELISA requires 100 µl of serum for one test and about 400 µl of serum for four tests. Therefore, the present diagnostic kit is convenient for children or infant. Furthermore, the ELISA method obtains the blood by vein puncture, which distresses the babies.
(5) The results of the detection will not be influenced instruments or the reaction environments.
(6) The operation is very simple because the color will appear immediately after the addition of the colloidal gold conjugates and the kit can be stored at room temperature for a long time.

The kit of the present invention utilizes colloidal immunogold filtration assay technique and realizes simultaneous detection of multiple antigens and antibodies on one carrier. It is easy to operate, rapid, accurate and suitable for detecting multiple diseases of a variety of blood samples, especially for large scaled blood screens. It also provides a new idea for the detection of infectious diseases.

### Brief description of the drawings

Fig. 1 is a schematic drawing of the sample spotted on the nitrocellulose membrane. In this figure, the reference numbers 1,2,3,4 indicate the positions of the samples respectively. The "C" between two spots (dots) indicates the position for the goat anti-mouse IgG antibody.
Fig. 2 is a schematic drawing of the detection results of No. 1 serum wherein the detection results are all negative.
Fig. 3 is a schematic drawing of the detection results of No. 2 serum wherein HCV is positive.
Fig. 4 is a schematic drawing of the detection results of No. 3 serum wherein the syphilitic antibody, HIV-1&2 antibodies are positive.
Fig. 5 is a schematic drawing of the detection results of No. 4 serum wherein the syphilitic antibody, HIV-1&2 antibodies and HBsAg antibody are positive.

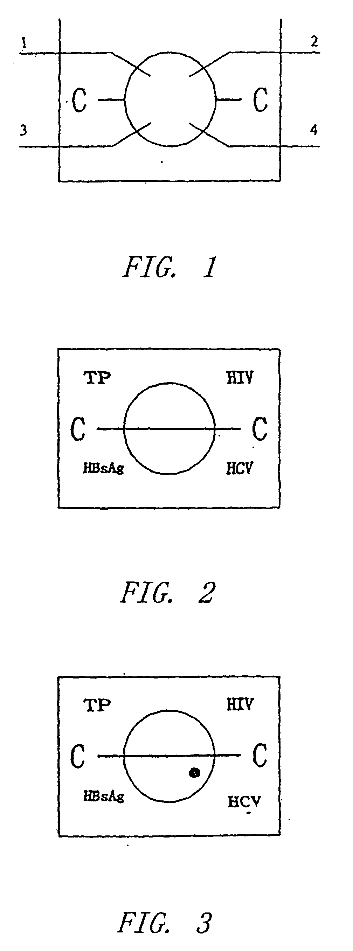

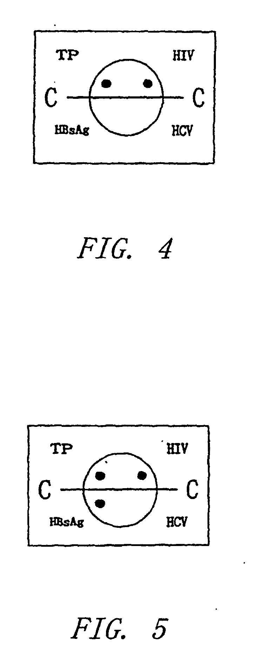

In the Figs 2-5, the "C-C", which represents goat anti-mouse IgG antibody, is positive.

### Examples

### A. Materials

In the preferred embodiments of the present invention, the following materials were used: The mouse anti-human HBsAg monoclonal antibodies (Mab) S1 and S2 were provided by Beierle Biotech Co. Ltd. The HCV antigen, TP47 syphilitic antigen, TP15 syphilitic antigen, the mixture of TP47/TP15 syphilitic antigens, HIV-1 antigen, HIV-2 antigen and the mixture of HIV-1/2 antigens were provided by BioDesign (Saco, Maine USA). The nitrocellulose membrane was provided by Schleicher & Schuell (Germany). The sample serum to be tested were provided by the Center for Disease Control of Shanghai.

### B. The immunogold filtration assay device was prepared as follows:

(1) The HBsAg monoclonal antibody S2 (mouse anti-human) was dissolved with PBS (0.02M, pH8.0) and dialyzed overnight at 4°C. Then, the HBsAg monoclonal antibody S2 was diluted to 0.5mg/ml with PBS (0.02M, pH8.0) and stored at 4°C for further use.
(2) The HCV antigen was dissolved with PBS (0.02M, pH8.0) and dialyzed overnight at 4°C. Then, the HCV antigen was diluted to 0.4 mg/ml with PBS (0.02M, pH8.0) and stored at 4°C for further use.
(3) The mixture of TP47/TP15 antigens was dissolved with PBS (0.02M, pH8.0) and dialyzed overnight at 4°C. Then, the mixture of TP47/TP15 antigens was diluted to 0.5 mg/ml with PBS (0.02M, pH8.0) and stored at 4°C for further use.
(4) The mixture of HIV-1/2 antigens was dissolved with PBS (0.02M, pH8.0) and dialyzed overnight at 4°C. Then, the mixture of HIV-1/2 antigens was diluted to 0.6mg/ml with PBS (0.02M, pH8.0) and stored at 4°C for further use.
(5) The goat anti-mouse IgG antibody was dissolved with PBS (0.02M, pH8.0) and dialyzed overnight at 4°C. Then, the goat anti-mouse IgG antibody was diluted to 0.6 mg/ml with PBS (0.02M, pH8.0) and stored at 4°C for further use.
(6) As shown in Fig. 1, four kind of proteins, i.e., the mixture of TP47/TP15 syphilitic antigens, the mixture of HIV-1/2 antigens, HBsAg monoclonal antibody S2 and HCV antigen, each in a volume of 1.0 µl, were dotted onto the positions 1, 2, 3, 4 on the nitrocellulose membrane by using micropipette. Then, the goat anti-mouse IgG antibody was sucked by a 0.2mm drawing pen and a line was drawn between two dots labeled "C" on the nitrocellulose membrane.
(7) The nitrocellulose membrane was dried in an oven at 37°C for 30 minutes and then left at room temperature for 25 minutes. The membrane was immersed in a blocking solution at 37°C for 20 minutes and subsequently washed and vibrated in a washing solution for 5 minutes at room temperature. After washing for several times, the membrane was air dried at room temperature.
(8) Two layers of water-absorbing filter paper were laid under the above nitrocellulose membrane. Both the nitrocellulose membranes and the filter paper were placed and fixed in the plastic reaction device.

### C. The buffer was a solution obtained by mixing 0.10M PBS (pH7.8) and 0.30% Tween20 in the same volume.

### D. The colloidal gold-labeled solution contains the colloidal gold-labeled HBsAg Mab S1 (mouse anti-human), the colloidal gold-labeled HCV antigen, the colloidal gold-labeled TP47/TP15 syphilitic antigens mixture, the colloidal gold-labeled HIV-1/2 antigens mixture in a ratio of 1 : I : 1.2-2.5 : 1.2-2.5. The colloidal gold particles of each colloidal gold conjugates had a mean particle size of approximately 30 nm. The method for preparing the colloidal gold conjugate was as follows:

### (1) preparing the colloidal gold-labeled HBsAg Mab S1 (mouse anti-human)

The HBsAg Mab S1 (mouse anti-human) was slowly added into the colloidal gold solution of which the gold particl diameter is 30 nm under magnetic stirring to a final concentration of 20 µg/ml. After stirring for 30 minutes at room temperature, 10% BSA solution was added to make the antibody a final concentration of 0.6% and the mixture was stirred for 5 minutes at room temperature. 10% PEG20000 solution was added to make the antibody a final concentration of 0.2% and the mixture was stirred for 5 minutes at room temperature. The mixture was centrifuged at 12000-15000 r/min for 60 minutes. Then the supernatant was discarded and the precipitate was dissolved in a storing solution. The solution was filtered through a 0.45 µm filter membrane and stored at 4°C for further use.

### (2) preparing the colloidal gold-labeled HCV antigen

The HCV antigen was slowly added into the colloidal gold solution of which the gold partical diameter is 30nm under magnetic stirring to a final concentration of 90 µg/ml. After stirring for 30 minutes at room temperature, 10% BSA solution was added to make the antigen a final concentration of 0.6% and the mixture was stirred for 5 minutes at room temperature. Then, 10% PEG20000 solution was added to make the antigen a final concentration of 0.2% and the mixture was stirred for 5 minutes at room temperature. The mixture was centrifuged at 12000-15000 r/min for 60 minutes. Then the supernatant was discarded and the precipitate was dissolved in a storing solution. The solution was filtered through a 0.45um filter membrane and stored at 4°C for further use.

### (3) preparing the colloidal gold-labeled mixture of the TP47/TP15 syphilitic antigens

The TP47 syphilitic antigen was slowly added into the colloidal gold solution of which the gold particle diameter is 30nm under magnetic stirring to a final concentration of 90 µg/ml. After stirring for 30 minutes at room temperature, 10% BSA solution was added to make the antigen a final concentration of 0.6% and the mixture was stirred for 5 minutes at room temperature. Then, 10% PEG20000 solution was added to make the antigen a final concentration of 0.2% and the mixture was stirred for 5 minutes at room temperature. The mixture was centrifuged at 12000-15000 r/min for 60 minutes. Then the supernatant was discarded and the precipitate was dissolved in a storing solution. The solution was filtered through a 0.45 µm filter membrane and stored at 4°C for further use.

The preparation of the colloidal gold-labeled TP15 antigen was the same as that of the TP47 conjugate.

The colloidal gold-labeled TP47 antigen was mixed with the same volume of the colloidal gold-labeled TP 15 antigen, and then stored at 4°C for further use.

### (4) preparing the colloidal gold-labeled HIV-1/2 antigen

The HIV-1 antigen was slowly added into the colloidal gold solution of which the gold particle diameter is 30nm to a final concentration of 100µg/ml under magnetic stirring. After stirring for 30 minutes at room temperature, 10% BSA solution was added to make the antigen a final concentration of 0.6% and the mixture was stirred for 5 minutes at room temperature. Then, 10% PEG20000 solution was added to make the antigen a final concentration of 0.2% and the mixture was stirred for 5 minutes at room temperature. The mixture was centrifuged at 12000-15000 r/min for 60 minutes. Then the supernatant was discarded and the precipitate was dissolved in a storing solution. The solution was filtered through a 0.45um filter membrane and stored at 4°C for further use.

The preparation of the colloidal gold-labeled HIV-2 antigen was the same as that of HIV-1 conjugate.

The colloidal gold-labeled HIV-1 antigen was mixed with the HIV-2 antigen in the same volume and stored at 4°C for further use.

When the HIV antigen in the colloidal gold-labeled solution was the mixture of several kinds of HIV antigens, each of the colloidal gold-labeled HIV antigens should be prepared separately, and then diluted in a certain ratio, mixed and stored for further use.

The analytical kits prepared as described above were used to test different sera. The results from the hospitals indicated that the provider of No.1 serum did not suffer from any of these four kinds of infectious diseases. The provider of No. 2 serum was infected with Hepatitis C. The provider of No. 3 serum was infected with syphilis and HIV. The provider No. 4 serum was infected with Hepatitis B, syphilis and HIV. Figs. 2, 3, 4, 5 showed the above detection results, respectively.

## Claims

1. A diagnostic kit for simultaneously detecting multiple infectious diseases consisting of an immunogold filtration assay device, buffer and the mixed solution of the colloidal gold conjugates,
wherein said immunogold filtration assay device comprises a nitrocellulose membrane with HBsAg monoclonal antibody, HCV antigen, syphilitic antigen, HIV antigen and goat anti-mouse IgG antibody for quality control dotted thereon separately;
wherein the mixed solution of the colloidal gold conjugates comprises four kinds of colloidal gold conjugates, including the colloidal gold conjugates of HBsAg monoclonal antibody, HCV antigen, syphilitic antigen and HIV antigen; the protein concentrations of the HBsAg monoclonal antibody, HCV antigen, syphilitic antigen and HIV antigen, when used to form the colloidal gold conjugates, are 20-50 µg/ml, 90-120 µg/ml, 90-120 µg/ml and 80-120 µg/ml, respectively; the diameter of the colloidal gold particles for preparing each colloidal gold conjugates is 20-30 nm; these four kinds of conjugates are mixed in a volume ratio of 1:1:1.2-2.5:1.2-2.5 to form said mixed solution of the colloidal gold conjugates.

2. The diagnostic kit for simultaneously detecting multiple infectious diseases according to claim 1, wherein said syphilitic antigen is a single syphilitic antigen or the mixture of multiple syphilitic antigens; said HIV antigen is HIV-1 antigen, HIV-2 antigen or the mixture of HIV-1 antigen and HIV-2 antigen.

3. The diagnostic kit for simultaneously detecting multiple infectious diseases according to claim 1, wherein said buffer is the PBS buffer comprising 0.03-0.05% Tween 20 at pH7.2-8.8.

4. A method for preparing the diagnostic kit for simultaneously detecting multiple infectious diseases according to claim 1, said method comprises the following steps:
A. preparing the analytical device for immunogold filtration assay, which comprises the following steps:
1). preparing the samples dotted onto the membrane, comprising dissolving the HBsAg monoclonal antibody, the HCV antigen, the syphilis antigen and the HIV antigen with PBS (0.02M, pH8.0), respectively; dialyzing overnight at 4°C; diluting each dialyzed proteins to 0.2-1.0 mg/ml with PBS (0.02M, pH8.0);
2). applying the samples onto the membrane, comprising
pipetting the above described HBsAg monoclonal antibody, HCV antigen, syphilitic antigen and HIV antigen and carefully dotting them onto the specific positions on the nitrocellulose membrane in a volume of 0.3-3 µl for each proteins; pipetting goat anti-mouse IgG antibody for labeling a mark, which may be a dot, a line or in other forms, at the position far from the above dotting positions on the same nitrocellulose membrane;
3). post-treating the membrane, comprising
drying the above nitrocellulose membrane in an oven at 37°C for 30 minutes; leaving it at room temperature for over 20 minutes; immersing it in a blocking solution at 37°C for 20 minutes; washing and vibrating in a washing solution for 5-10 minutes at room temperature; repeating the above washing step for several times; and air drying said membrane;
4). assembling the device, comprising
putting two layers of water-absorbing filter paper under the nitrocellulose membrane; placing and immobilizing both the membrane and the filter paper in the plastic reaction device;
B. preparing the mixed solution of the colloidal gold conjugates, which comprises the following steps:
1). preparing the colloidal gold particles, comprising
heating 0.01% chloroauric acid solution to the boiling point; adding 1% sodium citrate solution quickly; keeping the solution boiling for 5 minutes to make the size of the colloidal gold particles to be 20-30 nm;
2). preparing the colloidal gold conjugates, comprising
2.1). preparing the colloidal gold-labeled HBsAg monoclonal antibody, comprising the following procedures:
adding the HBsAg monoclonal antibody slowly into the colloidal gold solution, of which the particle diameter is, 20-30nm under magnetic stirring to a final concentration of 20-50µg/ml; stirring it for 30 minutes at room temperature; adding 10% BSA solution to make the antibody a final concentration of 0.2-1.0%; stirring the mixture for 5 minutes at room temperature; adding 10% PEG20000 solution to make the antibody a final concentration of 0.1-0.5%; stirring the mixture for 5 minutes at room temperature; centrifuging the mixture at 12000-15000r/min for 60 minutes; discarding the supernatant; dissolving the precipitate in a storing solution; filtering the solution through a 0.45 µm filter membrane; and storing the mixture at 4°C for further use;
2.2). preparing the colloidal gold-labeled HCV antigen, comprising the following procedures:
adding the HCV antigen slowly into the colloidal gold solution, of which the particle diameter is 20-30nm ,under magnetic stirring to a final concentration of 90-120µg/ml; stirring the mixture for 30 minutes at room temperature; adding 10% BSA solution to make the antigen a final concentration of 0.2-1.0%; stirring the mixture for 5 minutes at room temperature; adding 10% PEG20000 solution to make the antigen a final concentration of 0.1-0.5%; stirring the mixture for 5 minutes at room temperature; centrifuging the mixture at 12000-15000 r/min for 60 minutes; discarding the supernatant; dissolving the precipitate in a storing solution; filtering the solution through a 0.45 µm filter membrane; and storing the mixture at 4°C for further use;
2.3). preparing the colloidal gold-labeled syphilitic antigen, comprising the following procedures:
adding the syphilitic antigen slowly into the colloidal gold solution of which the particle diameter is 20-30nm, under magnetic stirring, to a final concentration of 90-120 µg/ml; stirring the mixture for 30 minutes at room temperature; adding 10% BSA solution to make the antigen a final concentration of 0.2-1.0%; stirring the mixture for 5 minutes at room temperature; adding 10% PEG20000 solution to make the antigen a final concentration of 0.1-0.5%; stirring the mixture for 5 minutes at room temperature; centrifuging the mixture at 12000-15000 r/min for 60 minutes; discarding the supernatant; dissolving the precipitate in a storing solution; filtering the solution through a 0.45 µm filter membrane; and storing the mixture at 4°C for further use;
2.4). preparing the colloidal gold-labeled HIV antigen comprising the following procedures:
adding the HIV antigen slowly into a colloidal gold solution of which the particle diameter is 20-30nm, under magnetic stirring, to a final concentration of 80-120 µg/ml; stirring the mixture for 30 minutes at room temperature; adding 10% BSA solution to make the antigen a final concentration of 0.2-1.0%; stirring the mixture for 5 minutes at room temperature; adding 10% PEG20000 solution to make the antigen a final concentration of 0.1-0.5%; stirring the mixture for 5 minutes at room temperature; centrifuging the mixture at 12000-15000 r/min for 60 minutes; discarding the supernatant; dissolving the precipitate in a storing solution; filtering the solution through a 0.45 µm filter membrane; and storing the mixture at 4°C for further use;
3). preparing the mixed solution of the colloidal gold conjugates by
mixing the colloidal gold conjugates separately prepared as above in the volume ratio of the HBsAg monoclonal antibody : HCV antigen : syphilitic antigen : HIV antigen being 1:1:1.2-2.5:1.2-2.5;
C. preparing the buffer by
adding Tween 20 into PBS (pH7.2-8.8) to a final concentration of 0.03-0.05%.

5. The use of the diagnostic kit according to claim 1 for simultaneously detecting Hepatitis B, Hepatitis C, syphilis and AIDS.

## Patentansprüche

1. Diagnosekit zur Simultandetektion mehrerer Infektionskrankheiten, welches aus einer Vorrichtung für ein Immunogold-Filtrationsassay, einem Puffer und einer vermengten Lösung kolloidaler Goldkonjugate besteht, wobei die Vorrichtung für das Immunogold-Filtrationsassay eine Nitrozellulosemembran aufweist, auf welcher ein HBsAg monoklonaler Antikörper, ein HCV Antigen, ein Syphilis-Antigen, ein HIV Antigen und zur Qualitätskontrolle ein Ziegen-Anti-Maus IgG Antikörper jeweils getrennt punktförmig aufgebracht vorliegen;
wobei die vermengte Lösung kolloidaler Goldkonjugate vier Arten von kolloidalen Goldkonjugaten umfasst, einschließlich der kolloidalen Goldkonjugate des HBsAg monoklonalen Antikörpers, des HCV Antigens, des Syphilis-Antigens und des HIV Antigens, wobei ferner die Proteinkonzentrationen des HBsAg monoklonalen Antikörpers, des HCV Antigens, des Syphilis-Antigens und des HIV Antigens zur Bildung der kolloidalen Goldkonjugate jeweils 20-50 µg/ml, 90-120 µg/ml, 90-120 µg/ml und 80-120 µg/ml betragen und der Durchmesser der kolloidalen Goldpartikel zur Herstellung des jeweiligen kolloidalen Goldkonjugats 20-30 nm ist und wobei ferner diese vier Arten der Konjugate in einem Volumenverhältnis von 1:1:1,2-2,5:1,2-2,5 vermengt werden, um die vermengte Lösung der kolloidalen Goldkonjugate zu bilden.

2. Diagnosekit zur Simultandetektion mehrfacher Infektionskrankheiten nach Anspruch 1, wobei das Syphilis-Antigen ein einzelnes Syphilis-Antigen oder eine Mischung aus mehreren Syphilis-Antigenen ist und wobei ferner das HIV-Antigen das HIV-1 Antigen, das HIV-2 Antigen oder eine Mischung aus dem HIV-1 und dem HIV-2 Antigen ist.

3. Diagnosekit zur Simultandetektion mehrerer Infektionskrankheiten nach Anspruch 1, wobei der Puffer ein PBS-Puffer mit 0,03-0,05 % Tween 20 mit einem pH-Wert von 7,2-8,8 ist.

4. Verfahren zur Herstellung des Diagnosekits zur Simultandetektion mehrerer Infektionskrankheiten nach Anspruch 1, wobei das Verfahren die folgenden Schritte aufweist:
A. Herstellen der analytischen Vorrichtung für das Immunogold-Filtrationsassay, das die folgenden Schritte aufweist:
1.) Herstellen der auf die Membran punktförmig aufzubringenden Proben, mit den Schritten:
Lösen des HBsAg monoklonalen Antikörpers, des HCV Antigens, des Syphilis-Antigens und des HIV Antigens jeweils mit PBS (0,02M, pH 8,0); Dialysieren über Nacht bei 4°C, Verdünnen jedes dialysierten Proteins auf 0,2-1,0 mg/ml mit PBS (0,02M, pH 8,0);
2.) Auftragen der Proben auf die Membran, mit den Schritten:
Pipettieren des wie oben beschriebenen HBsAg monoklonalen Antikörpers, des HCV Antigens, des Syphilis-Anitgens und des HIV Antigens und vorsichtiges punktförmiges Auftragen auf die spezifischen Positionen auf der Nitrozellulosemembran, mit einem Volumen von 0,3-3 µl je Protein; Pipettieren eines Ziegen-Anti-Maus IgG-Antikörpers zum Markieren eines Bezugspunkts, welcher als Punkt, als Linie oder in anderen Formen vorliegen kann, und zwar auf eine Position, die von den oben angegebenen punktförmig aufgebrachten Positionen auf der gleichen Nitrozellulosemembran weit entfernt ist;
3.) Nachbehandeln der Membran mit den Schritten:
Trocknen der oben beschriebenen Nitrozellulosemembran in einem Ofen bei 37°C für 30 Minuten; Stehenlassen bei Raumtemperatur für mehr als 20 Minuten; Eintauchen der Nitrozellulosemembran in eine Blockierungslösung bei 37°C für 20 Minuten; Waschen und Schütteln in einer Waschlösung für 5-10 Minuten bei Raumtemperatur; mehrmaliges Wiederholen des oben angegeben Waschschritts und Lufttrocknen der Membran;
4.) Zusammenbau der Vorrichtung mit den Schritten
Anbringen von zwei Lagen eines wasseraufnehmenden Filterpapiers unter die Nitrozellulosemembran; Plazieren und Immobilisieren sowohl der Membran als auch der Filterpapiere in einer Plastikreaktionsvorrichtung;
B. Herstellen der vermengten Lösung der kolloidalen Goldkonjugate, mit den folgenden Schritten:
1.) Herstellen der kolloidalen Goldpartikel, mit den Schritten:
Erhitzen einer 0,01%igen Goldchlorwasserstoffsäure-Lösung auf den Siedepunkt; rasches Hinzufügen einer 1%igen Natriumcitrat-Lösung; Kochenlassen der Lösung für 5 Minuten, um die Größe der kolloidalen Goldpartikel auf 20-30 nm einzustellen;
2.) Herstellen der kolloidalen Goldkonjugate mit den Schritten:
2.1 Herstellen des kolloidalen gold-markierten HBsAg monoklonalen Antikörpers mit den folgenden Verfahren:
langsames Hinzufügen des HBsAg monoklonalen Antikörpers in die kolloidale Goldlösung, deren Partikeldurchmesser 20-30 nm beträgt, unter Rühren mit einem Magnetrührer auf eine Endkonzentration von 20-50 µg/ml; Rühren der Mischung für 30 Minuten bei Raumtemperatur; Hinzufügen einer 10%igen BSA-Lösung um den Antikörper auf eine Endkonzentration von 0,2-1,0 % zu bringen; Rühren der Mischung für 5 Minuten bei Raumtemperatur; Hinzfügen einer 10%igen PEG20000-Lösung, um den Antikörper auf eine Endkonzentration von 0,1-0,5 % zu bringen; Rühren der Mischung für 5 Minuten bei Raumtemperatur; Zentrifugieren der Mischung mit 12000-15000 r/min für 60 min; Verwerfen des Überstandes; Auflösen des Präzipitats in einer Aufbewahrungslösung, Filtern der Lösung durch eine 0,45 µm Filtermembran und Aufbewahren der Mischung bei 4°C für den weiteren Gebrauch;
2.2 Herstellen des kolloidalen gold-markierten HCV Antigens mit den folgenden Verfahren:
langsames Hinzufügen des HCV Antigens in die kolloidale Goldlösung, deren Partikeldurchmesser 20-30 nm beträgt, unter Rühren mit einem Magnetrührer auf eine Endkonzentration von 90-120 µg/ml; Rühren der Mischung für 30 Minuten bei Raumtemperatur; Hinzufügen einer 10%igen BSA-Lösung, um das Antigen auf eine Endkonzentration von 0,2-1,0 % zu bringen; Rühren der Mischung für 5 Minuten bei Raumtemperatur; Hinzufügen einer 10%igen PEG20000-Lösung, um das Antigen auf eine Endkonzentration von 0,1-0,5 % zu bringen; Rühren der Mischung für 5 Minuten bei Raumtemperatur; Zentrifugieren der Mischung mit 12000-15000 r/min für 60 Minuten; Verwerfen des Überstandes; Auflösen des Präzipitats in einer Aufbewahrungslösung; Filtern der Lösung durch eine 0,45 µm Filtermembran und Aufbewahren der Mischung bei 4°C für den weiteren Gebrauch;
2.3 Herstellen des kolloidalen gold-markierten Syphilis-Antigens mit den folgenden Verfahren:
langsames Hinzufügen des Syphilis-Antigens in die kolloidale Goldlösung, deren Partikeldurchmesser 20-30 nm beträgt, unter Rühren mit einem Magnetrührer auf eine Endkonzentration von 90-120 µg/ml; Rühren der Mischung für 30 Minuten bei Raumtemperatur; Hinzufügen einer 10%igen BSA-Lösung, um das Antigen auf eine Endkonzentration von 0,2-1,0 % zu bringen; Rühren der Mischung für 5 Minuten bei Raumtemperatur; Hinzufügen einer 10%igen PEG20000-Lösung, um das Antigen auf eine Endkonzentration von 0,1-0,5 % zu bringen; Rühren der Mischung für 5 Minuten bei Raumtemperatur; Zentrifugieren der Mischung mit 12000-15000 r/min für 60 Minuten; Verwerfen des Überstandes; Auflösen des Präzipitats in einer Aufbewahrungslösung; Filtern der Lösung durch eine 0,45 µm Filtermembran und Aufbewahren der Mischung bei 4°C für den weiteren Gebrauch;
2.4 Herstellen des kolloidalen gold-markierten HIV Antigens mit den folgenden Verfahren:
langsames Hinzufügen des HIV Antigens in eine kolloidale Goldlösung, deren Partikeldurchmesser 20-30 nm beträgt, unter Rühren mit einem Magnetrührer auf eine Endkonzentration von 80-120 µg/ml; Rühren der Mischung für 30 Minuten bei Raumtemperatur; Hinzufügen einer 10%igen BSA-Lösung, um das Antigen auf eine Endkonzentration von 0,2-1,0 % zu bringen; Rühren der Mischung für 5 Minuten bei Raumtemperatur; Hinzufügen einer 10%igen PEG20000-Lösung, um das Antigen auf eine Endkonzentration von 0,1-0,5 % zu bringen; Rühren der Mischung für 5 Minuten bei Raumtemperatur; Zentrifugieren der Mischung mit 12000-15000 r/min für 60 Minuten; Verwerfen des Überstandes; Auflösen des Präzipitats in einer Aufbewahrungslösung; Filtern der Lösung durch eine 0,45 µm Filtermembran und Aufbewahren der Mischung bei 4°C für den weiteren Gebrauch;
3.) Herstellen der vermengten Lösung der kolloidalen Goldkonjugate durch:
Vermengen der wie oben getrennt hergestellten kolloidalen Goldkonjugate in einem Volumenverhältnis des HBsAg monoklonalen Antikörpers: HCV Antigen: Syphilis-Antigen: HIV Antigen von 1:1:1,2-2,5:1,2-2,5;
C. Herstellen des Puffers durch
Hinzufügen von Tween 20 zu PBS (pH 7,2-8,8) auf eine Endkonzentration von 0,03-0,05 %.

5. Verwendung des Diagnosekits nach Anspruch 1 zur Simultandetektion von Hepatitis B, Hepatitis C, Syphilis and AIDS.

## Revendications

1. Kit de diagnostic pour la détection simultanée de multiples maladies infectieuses, composé d'un dispositif d'essai par filtration d'immunogold, de tampon et de la solution mixte des conjugués d'or colloïdal,
dans lequel ledit dispositif d'essai par filtration d'immunogold comprend une membrane en nitrocellulose sur laquelle sont déposés séparément par points un anticorps monoclonal anti-HBsAg, un antigène HCV, un antigène syphilitique, un antigène HIV et un anticorps de chèvre anti-IgG de souris pour le contrôle de qualité ;
dans lequel la solution mixte des conjugués d'or colloïdal comprend quatre sortes de conjugués d'or colloïdal, comprenant les conjugués d'or colloïdal de l'anticorps monoclonal anti-HBsAg, de l'antigène HCV, de l'antigène syphilitique et de l'antigène HIV ; les concentrations en protéines de l'anticorps monoclonal anti-HBsAg, de l'antigène HCV, de l'antigène syphilitique et de l'antigène HIV, lorsqu'ils sont utilisés pour former les conjugués d'or colloïdal, sont respectivement de 20 à 50 µg/ml, de 90 à 120 µg/ml, de 90 à 120 µg/ml et de 80 à 120 µg/ml ; le diamètre des particules d'or colloïdal servant à préparer chaque conjugué d'or colloïdal est de 20 à 30 nm ; ces quatre sortes de conjugués sont mélangées selon un rapport en volume de 1:1:1,2-2,5:1,2-2,5 afin de former ladite solution mixte des conjugués d'or colloïdal.

2. Kit de diagnostic pour la détection simultanée de multiples maladies infectieuses selon la revendication 1, dans lequel ledit antigène syphilitique est un antigène syphilitique simple ou le mélange de multiples antigènes syphilitiques ; ledit antigène HIV est l'antigène HIV-1, l'antigène HIV-2 ou le mélange de l'antigène HIV-1 et de l'antigène HIV-2.

3. Kit de diagnostic pour la détection simultanée de multiples maladies infectieuses selon la revendication 1, dans lequel ledit tampon est le tampon PBS comprenant de 0,03 à 0,05% de Tween 20 à un pH de 7,2 à 8,8.

4. Procédé de préparation du kit de diagnostic pour la détection simultanée de multiples maladies infectieuses selon la revendication 1, ledit procédé comprenant les étapes suivantes :
A. Préparation du dispositif d'analyse pour l'essai de filtration d'immunogold, qui comprend les étapes suivantes :
1) Préparation des échantillons déposés par points sur la membrane, comprenant :
dissoudre l'anticorps monoclonal anti-HBsAg, l'antigène HCV, l'antigène syphilitique et l'antigène HIV, respectivement, avec du PBS (0,02 M, pH 8,0) ; dialyser pendant une nuit à 4°C ; diluer chacune des protéines dialysées à 0,2 à 1,0 mg/ml avec du PBS (0,02 M, pH 8,0) ;
2) Dépôt des échantillons sur la membrane, comprenant :
pipeter l'anticorps monoclonal anti-HBsAg, l'antigène HCV, l'antigène syphilitique et l'antigène HIV décrits ci-dessus et les déposer avec précautions par points dans des positions spécifiques sur la membrane en nitrocellulose en un volume de 0,3 à 3 µl pour chacune des protéines ; pipeter de l'anticorps de chèvre anti-IgG de souris afin de marquer un repère, qui peut être un point, une ligne ou d'autres formes, dans une position éloignée des positions de dépôt de points ci-dessus sur la même membrane en nitrocellulose ;
3) Post-traitement de la membrane, comprenant :
sécher la membrane en nitrocellulose ci-dessus dans une étuve à 37°C pendant 30 minutes ; la laisser à la température ambiante pendant plus de 20 minutes ; la plonger dans une solution de blocage à 37°C pendant 20 minutes ; laver et vibrer dans une solution de lavage pendant 5 à 10 minutes à la température ambiante ; répéter l'étape de lavage ci-dessus à plusieurs reprises ; et sécher à l'air ladite membrane ;
4) Assemblage du dispositif, comprenant :
mettre deux couches de papier filtre absorbant l'eau sous la membrane en nitrocellulose ; placer et immobiliser à la fois la membrane et le papier filtre dans le dispositif réactionnel en plastique ;
B. Préparation de la solution mixte des conjugués d'or colloïdal, qui comprend les étapes suivantes :
1) Préparation des particules d'or colloïdal, comprenant :
chauffer une solution d'acide chloraurique à 0,01% au point d'ébullition ; ajouter rapidement une solution de citrate de sodium à 1% ; maintenir la solution à ébullition pendant 5 minutes pour amener la taille des particules d'or colloïdal dans la plage de 20 à 30 nm ;
2) Préparation des conjugués d'or colloïdal, comprenant :
2.1) Préparation de l'anticorps monoclonal anti-HBsAg marqué à l'or colloïdal, comprenant les opérations suivantes :
ajouter lentement l'anticorps monoclonal anti-HBsAg dans la solution d'or colloïdal dont le diamètre de particules est de 20 à 30 nm sous agitation magnétique à une concentration finale de 20 à 50 µg/ml ; l'agiter pendant 30 minutes à la température ambiante ; ajouter une solution de SAB à 10% de manière à obtenir une concentration finale en anticorps de 0,2 à 1,0% ; agiter le mélange pendant 5 minutes à la température ambiante ; ajouter une solution de PEG 20000 à 10% de manière à obtenir une concentration finale en anticorps de 0,1 à 0,5% ; agiter le mélange pendant 5 minutes à la température ambiante ; centrifuger le mélange à 12000-15000 tr/min pendant 60 minutes ; jeter le surnageant ; dissoudre le précipité dans une solution de stockage ; filtrer la solution à travers une membrane de filtration de 0,45 µm ; et stocker le mélange à 4°C en vue d'une utilisation ultérieure ;
2.2) Préparation de l'antigène HCV marqué à l'or colloïdal, comprenant les opérations suivantes :
ajouter lentement l'antigène HCV dans la solution d'or colloïdal dont la taille de particules est de 20 à 30 nm sous agitation magnétique à une concentration finale de 90 à 120 µg/ml ; agiter le mélange pendant 30 minutes à la température ambiante ; ajouter une solution de SAB à 10% de manière à obtenir une concentration finale en antigène de 0,2 à 1,0% ; agiter le mélange pendant 5 minutes à la température ambiante ; ajouter une solution de PEG 20000 à 10% de manière à obtenir une concentration finale en antigène de 0,1 à 0,5% ; agiter le mélange pendant 5 minutes à la température ambiante ; centrifuger le mélange à 12000-15000 tr/min pendant 60 minutes ; jeter le surnageant ; dissoudre le précipité dans une solution de stockage ; filtrer la solution à travers une membrane de filtration de 0,45 µm ; et stocker le mélange à 4°C en vue d'une utilisation ultérieure ;
2.3) Préparation de l'antigène syphilitique marqué à l'or colloïdal, comprenant les opérations suivantes :
ajouter lentement l'antigène syphilitique dans la solution d'or colloïdal dont le diamètre est de 20 à 30 nm sous agitation magnétique à une concentration finale de 90 à 120 µg/ml ; agiter le mélange pendant 30 minutes à la température ambiante ; ajouter une solution de SAB à 10% de manière à obtenir une concentration finale en antigène de 0,2 à 1,0% ; agiter le mélange pendant 5 minutes à la température ambiante ; ajouter une solution de PEG 20000 à 10% de manière à obtenir une concentration finale en antigène de 0,1 à 0,5% ; agiter le mélange pendant 5 minutes à la température ambiante ; centrifuger le mélange à 12000-15000 tr/min pendant 60 minutes ; jeter le surnageant ; dissoudre le précipité dans une solution de stockage ; filtrer la solution à travers une membrane de filtration de 0,45 µm ; et stocker le mélange à 4°C en vue d'une utilisation ultérieure ;
2.4) Préparation de l'antigène HIV marqué à l'or colloïdal, comprenant les opérations suivantes :
ajouter lentement l'antigène HIV dans une solution d'or colloïdal dont le diamètre de particules est de 20 à 30 nm sous agitation magnétique à une concentration finale de 80 à 120 µg/ml ; agiter le mélange pendant 30 minutes à la température ambiante ; ajouter une solution de SAB à 10% de manière à obtenir une concentration finale en antigène de 0,2 à 1,0% ; agiter le mélange pendant 5 minutes à la température ambiante ; ajouter une solution de PEG 20000 à 10% de manière à obtenir une concentration finale en antigène de 0,1 à 0,5% ; agiter le mélange pendant 5 minutes à la température ambiante ; centrifuger le mélange à 12000-15000 tr/min pendant 60 minutes ; jeter le surnageant ; dissoudre le précipité dans une solution de stockage ; filtrer la solution à travers une membrane de filtration de 0,45 µm ; et stocker le mélange à 4°C en vue d'une utilisation ultérieure ;
3) Préparation de la solution mixte des conjugués d'or colloïdal en
mélangeant séparément les conjugués d'or colloïdal préparés ainsi que décrit ci-dessus selon un rapport en volume anticorps monoclonal anti-HBsAg : antigène HCV : antigène syphilitique : antigène HIV de 1:1:1,2-2,5:1,2-2,5 ;
C. Préparation du tampon en
ajoutant du Tween 20 dans du PBS (pH 7,2 à 8,8) à une concentration finale de 0,03 à 0,05%.

5. Utilisation du kit de diagnostic selon la revendication 1 pour détecter simultanément l'hépatite B, l'hépatite C, la syphilis et le SIDA.
